# EUROPEAN PATENT SPECIFICATION

(11) **EP 1 940 353 B1**
(45) Date of publication and mention of the grant of the patent: **24.11.2010**
(21) Application number: 06805904.7
(22) Date of filing: 27.09.2006
(51) Int. Cl.: A61K 9/08, A61K 47/32, A61K 31/7072, A61P 27/02

(54) **PHOTOSTABLE PHARMACEUTICAL COMPOSITION CONTAINING BRIVUDINE FOR THE TREATMENT OF HERPETIC KERATITIS**
PHOTOSTABILE PHARMAZEUTISCHE ZUSAMMENSETZUNG MIT BRIVUDIN ZUR BEHANDLUNG VON HERPETISCHER KERATITIS
COMPOSITION PHARMACEUTIQUE PHOTOSTABLE CONTENANT DE LA BRIVUDINE POUR LE TRAITEMENT DE LA KERATITE HERPETIQUE

(30) Priority: 29.09.2005 DE 102005046769
(43) Date of publication of application: 09.07.2008
(73) Proprietor: BERLIN-CHEMIE AG, 12489 Berlin (DE)
(72) Inventor: WIHSMANN, Marc, 15732 Schulzendorf (DE); SCHMITZ, Reinhard, 14055 Berlin (DE)
(74) Representative: Minoja, Fabrizio
(86) International application number: PCT/EP2006/009383
(87) International publication number: WO 2007/039201

(56) References cited:
- WO-A-02/056913
- WO-A-03/051375
- JOHNE C ET AL: "Herstellung und Analytik von Brivudin-Augentropfen 0,1%" KRANKENHAUSPHARMAZIE, DEUTSCHER APOTHEKER VERLAG, STUTTGART, DE, vol. 23, no. 4, 2002, page 174, XP008080972 ISSN: 0173-7597 cited in the application
- MAUDGAL P C ET AL: "Comparative evaluation of BVDU ((E)-5-(2-bromovinyl)-2'-deoxyuridine) in thetreatment of experimental herpes simplex keratitis in rabbits" BULLETIN DE LA SOCIETE BELGE D'OPHTALMOLOGIE, BRUXELLES, BE, vol. 186, 1979, pages 109-118, XP008080995 ISSN: 0081-0746
- MAUDGAL P C ET AL: "Bromovinyldeoxyuridine treatment of herpetic keratitis clinically resistant to other antiviral agents" CURRENT EYE RESEARCH, IRL PRESS, OXFORD, GB, vol. 10 Suppl, 1991, pages 193-199, XP008080989 ISSN: 0271-3683
- DE CLERCQ ERIK: "(E)5-(2-bromovinyl)-2'-deoxyuridine (BVDU)" MEDICINAL RESEARCH REVIEWS, vol. 25, no. 1, January 2005 (2005-01), pages 1-20, XP002442047 ISSN: 0198-6325

## Description

### FIELD OF THE INVENTION

This invention relates to pharmaceutical compositions containing the antiviral brivudine, which are useful for the treatment of ocular inflammatory conditions, particularly stromal keratitis induced by Herpes Simplex Virus Type 1 (HSV-1) or by Varicella-Zoster Herpes Vitus (VZV). The compositions allow photo protection of the antiviral with excellent tolerability and long-lasting delivery of brivudine.

### BACKGROUND OF THE INVENTION

Inflammatory diseases of the eye can be initiated by viral, bacterial, fungal, or parasitic infection and by autoimmunity. One particularly damaging kind of ocular inflammatory condition is the keratitis induced by HSV-1 or less commonly by HSV type 2. Corneal inflammation due to HSV is particularly severe and can persist for years even when treated. HSV keratitis can confine to the outer epithelial layer of the cornea (epithelial keratitis) causing corneal ulcers with the potential to penetrate into deeper structures of the cornea (stromal keratitis) which may lead to corneal necrosis (necrotising stromal keratitis). A common sequela is corneal vascularization and scarring. Ocular complications of herpes zoster ophthalmicus, caused by Varicella Zoster Virus (VZV), may comprise non-specific conjunctivitis, dendritiform epithelial or stromal keratitis, anterior keratouveitis, retinal hemorrhage or retinitis and may also affect the optic nerve.

Currently, HSV induced epithelial keratitis is treated with antivirals while HSV stromal keratitis is treated by topically applied corticosteroids with an antiviral cover (See, e.g., Wilhelmus, K.R. et al., Ophthalmology. 101:1883, 1994). However, corticosteroid therapy may prolong and possibly worsen the disease as well as introduce other effects such as enhancement of viral replication, cataracts, glaucoma, corneal melting, secondary infection, and corticosteroid dependence. (See Liesegang, T.J., Mayo Clin. Proc. 63:1092, 1988).

Pharmaceutical compositions containing an antiviral agent such as acyclovir, for ocular topical treatments, are described in several patents:
CN1342651 (CAplus AN: 2003:394238) describes the preparation, among others, of eye drop containing ganciclovir sodium dihydrate.
RU2158591 (CAplus, AN: 2002:13230) deals with the treatment of herpetic eye diseases , in particular, herpetic keratitis, with acyclovir 0.1 g in 10 ml soln. of lacrisin.

In WO2000004884 topical treatment of herpetic keratitis was achieved with a lotion containing acyclovir and adenosine.

JP10287552 (Caplus, AN 1998:693384) describes eye drop with an acyclovir suspension.

WO96/24367 relates to a pharmaceutical composition for the treatment of herpes simplex virus infections, comprising a quaternary ammonium compound as a first component.

An antiviral agent which appears to be very promising, even in the treatment of herpetic keratitis, is brivudine. (E) -5-(2-bromovinyl)-2'-deoxyuridine. Its *in vitro* efficacy against VZV is superior to both acyclovir and penciclovir [mean IC₅₀ in 13 clinical VZV strains: 0.001 µg/ml (brivudine), 0.2 µg/ml (acyclovir), and 0.91 µg/ml (penciclovir)] (Andrei G., Snoeck R., Reymen D. Eur. J. Clin. Microbiol. Infect.; 14; 318-319; 1995).

Furthermore, brivudine is a potent inhibitor of HSV-1 replication with a superior *in vitro* efficacy compared to acyclovir and penciclovir: in a panel of 23 clinical HSV-1 strains, brivudine proved to be almost twice as effective as acyclovir [mean IC₅₀: 0.52 µg/ml (brivudine) vs. 0.92 µg/ml (acyclovir)] (Andrei G., Snoeck R., Goubau P. Eur. J. Clin. Microbiol. Infect.; 11; 143-151; 1992 ). In two similar assays in a panel of 20 clinical isolates penciclovir showed a mean IC₅₀ of 0.6 µg/ml and 0.8 µg/ml (Weinberg A., Bate B.J., Masters H.B. Antimicrob. Agents Chemother.; 36; 2037-2038; 1992).

Earlier investigations predominantly in HSV-1 laboratory strains demonstrate an even lower IC₅₀ of brivudine between 0.004 µg/ml and 0.2 µg/ml (depending on the viral strain and the cell system) (De Clercq E., Descamps J., Verhelst G., J. Infect. Dis.; 141; 563-574; 1980; De Clercq E., Antimicrob. Agents Chemother.; 21; 661-663; 1982).

The use of brivudine for the treatment of ocular viral diseases was described in several animal and clinical tests: e.g. Antiviral Research (1984 oct) 4(5) 281-291; Current eye Research. 1991 10 suppl, 193-9, but no commercial pharmaceutical composition for topical ocular use has so far appeared on the market, suggesting that some problems have still to be solved.

Brivudine, in fact, has revealed particularly unstable in the conditions normally used for topical preparations. In those conditions brivudine was found to undergo an extensive photodegradation, which reduces the concentration of the active principle, decreasing the ultimate effect of the treatment and determining a significant local irritation that can discourage from using the pharmaceutical composition.

Since promising drugs for the treatment of herpetic keratitis are not yet available (exception is acyclovir, but it is toxic in the epithelium) short term brivudine formulations are used in selected hospitals (see Krankenhauspharmazie 2002, 23, 174), but also these brivudine formulations are characterised by a rapid lost of activity due to the degradation of the active.

In WO02/056913 topical pharmaceutical formulations (not for ocular uses) containing brivudine have been described: the antiviral agent was here stabilised with a high percentage of a metal oxide pigment, preferably TiO₂.

When pigments are used in a pharmaceutical formulations they are normally part of a water in oil (W/O) resp. oil in water (O/W) suspension or -exceptionally- of a slurry; in these systems the pigments are still solid.

Since photo protection of brivudine needs amounts of pigment not less than 20% the viscosity of the formulation is very high. Considering that the sickened eye is much more sensitive towards outer influences, an ophthalmic use of this kind of formulation would generate aches and pains.

In a quite similar way, WO03051375 describes other topical formulations where the active compound, brivudine, was stabilized against photo degradation with an UV-filter, in particular with one selected in the group of derivatives of o-hydroxy-benzophenone.

The invented solutions are not suitable for ocular formulation, since chemical UV-filters are dissolved within the formulation. In this form this substances are to be expected as eye-irritant. The sickened eye is markedly more sensitive and a remarkable protecting effect of the sunscreens is to be observed at concentrations higher than 5%. Therefore the use of dissolved sunscreens should be avoided.

### DESCRIPTION OF THE INVENTION

It has been surprisingly found that ophthalmic composition containing film-forming agents selected from polyvinyl pyrrolidones (PVP), polyvinyl alcohol (PVA) and polyacrylates (PA) prevent brivudine photodegradation allowing at the same time excellent tolerability and long-lasting local delivery of the drug.

In particular, it was observed that the film forming agents according to the invention ensure recovery rates of more than 85% brivudine (see Table 1), after irradiation with 8.9 J/cm², in contrast to other thickeners, including alginats, xanthane gum, carrageenan, acacia gum, guar gum, cellulose derivatives and agar gel (best observed result was 70 % recovery of brivudine after irradiation).

Formulations based on film formers are commonly used as artificial tear drops. They are approved since decades without any general or special adverse effects published. In additional studies performed by us to investigate the influence of brivudine on the drug product-tolerability, no deterioration of the pharmaceutical properties was observed. These studies also showed that brivudine remains for more than 2 hours in the ocular region without systemic absorption in the blood.

The invention therefore solves the problem of providing a brivudine-based ophthalmic product characterized by a low content of active substance and a noticeably increased stability against light-induced degradation in comparison with known formulations (without stabilising agent). In addition to the active substance, the composition may contain excipients, adjuvants, carriers, solubilisers and especially preservatives.

The composition of the invention is an ophthalmic composition containing from 0.05 to 0.3% w/w of brivudine as active ingredient and a film-forming agent selected from the group consisting of polyvinyl pyrrolidone (PVP), polyvinyl alcohol (PVA) and polyacrylate (PA).

According to a preferred embodiment, the ophthalmic pharmaceutical formulation contains 0.05 to 0.3% by weight of brivudine, 0.5 to 10% by weight of film forming agents, 0 to 2.0% by weight of preservative and a residual content of up to 100% by weight of pharmaceutically acceptable excipients, adjuvants, carriers, solubilisers.

In a particularly preferred embodiment of the invention, the ophthalmic formulation contains, besides the active principle, a film forming agent selected from the group consisting of PVP, PVA and polyacrylates, a preservative selected from the group consisting of chlorhexidine, chlorbutanol and boric acid, an antioxidant selected from the group consisting of butylated hydroxytoluene (BHT), esters of gallic acid, esters of ascorbic acid and esters of α-tocopherole. Other suitable excipients include flavours, buffers, surfactants and co-solvents.

The brivudine content ranges from 0.05 to 0.3% by the weight of the composition.

The amount of film former is preferably from 0.5 to 10%, more preferably from 1 to 5% by weight of the composition.

The amount of preservatives - if present - is preferably up to 2%, more preferably from 0.01 to 1.5% by weight of the composition.

The amount of antioxidants - if present - is preferably from 0.001 to 1%, more preferably from 0.001 to 0.1 % by weight of the composition.

The formulations of this invention may contain further carriers/enhancers and adjuvants such as water, monovalent alcohols such as ethanol, colorants, thickeners, plasticizers, surfactants, polyols, esters, electrolytes, gel forming agent, polar and non-polar oils, polymers, copolymers, emulsifiers, emulsion stabilisers.

In addition, liposomes can be used to improve the delivery of the active principle.

Further adjuvants and active substances may be present, including for example vitamin A or vitamin-A derivatives; vitamin E or vitamin-E derivatives; complex vitamins coloured or uncoloured plant extracts.

The preparation of the invention can be in the form of drops, solutions, gels or ointments, packaged in single dose or multiple dose containers.

A typical ocular preparation according to the present invention contains (%w/w):
0.1-0.3% of brivudine
1-7% of a film forming agent
0-2% of one or more preserving agents
0-2% buffer
0-0.9% NaCl
0-4% 0.1n NaOH ( up to pH ranging from 5.5 to 6.5)
0-25% surfactant
0-0.003% antioxidant
0.01-0.05% Sodium-EDTA
ad 100%water

The manufacturing is performed in an aseptic area under sterile conditions. The formulation is prepared using well known methods (mixing, stirring, homogenising, filtering) and with well known instruments (homogenisers e.g. Ultra Turrax, Silverson mixer or similar devices, sterile filters) - see, e.g., Remington's Pharmaceutical Sciences, and U.S. Pharmacopeia and Voigt, Rudolf, Pharmazeutische Technologie für Studium und Beruf, 7th revised edition, Berlin (1993).

Ophthalmic formulations containing Brivudine can be used to treat several ocular pathologies, in particular HSV-epithelial keratitis, HSV-stromal keratitis, ocular complications of herpes zoster ophthalmicus.

The effective amount of active ingredient may vary depending on factors such as the condition to be treated, the progression of the disease, the overall health conditions of the patient, the form, route and dose of administration and the severity of side effects. As used herein, the phrase "therapeutically effective dose" means a dose sufficient to ameliorate a symptom or sign of ocular inflammation. The appropriate doses can be easily determined using methods known in the art.

In appropriate circumstances, the composition according to the invention can be used for combined therapy. Preferably, the combined preparation will contain brivudine and an active ingredient selected from steroids, particularly prednisolone acetate and fluorometholone, and non-steroidal antinflammatory agents such as flurbiprofen, for simultaneous, sequential or separate administration.

The invention is further illustrated by the following non-limiting examples.

### Examples

Unless otherwise specified, the classes or groups of compounds mentioned throughout the patent description and the examples should be intended as follows:
*Polyvinyl pyrrolidones*: compounds known under the INCI names 1-Ethenyl-2-pyrrolidinone homopolymer and PVP and the marked name Kollidon (BASF), e. g. Kollidon 12 (molecular weight appr. 2,000-3,000), Kollidon 17 (molecular weight appr. 7,000-11,000), Kollidon 25 (molecular weight appr. 28,000-34,000) and (Kollidon 30 (molecular weight appr. 44,000-54,000), Kollidon 90 (molecular weight appr. 1,000,000-1,500,000); all qualities correspond to pharmaceutical Pharmacopoeias, see Ph.Eur. 01/2005:0685.
*Polyvinyl alcohols*: compounds known under the INCI names Ethenol homopolymer, PVA and the market name e.g. Kollicoat^{®} Protect (BASF); e.g. PVA 72,000 or PVA 145,000; all used qualities correspond to pharmaceutical Pharmacopoeias, see Ph.Eur. 01/2005:1961.
*Polyacrylates*: compounds known under INCI names polyacrylic acid and the market name e.g. Carbopol 934P, 941, 971, 940 and 980 or Carbopol ETD 2001, 2020 and 2050, resp. Noveon AA-1; qualities used correspond to pharmaceutical Pharmacopoeias, Ph.Eur. (see 01/2005:1299).
*Poloxamers*: synthetic block copolymers of ethylene oxide and propylene oxide. In principal all poloxamers described in European Pharmacopoeia (P 124, 188, 237, 338 and 407) can be used. The following types were found to be usable in particular: Poloxamer 407 (average molecule mass: 9840 - 14600) and Poloxamer 188 (average molecule mass: 7680 - 9510), see Ph.Eur. 01/2005:1464.

### Example 1

The following eye drops formulation was prepared under aseptic conditions by mixing the ingredients at a temperature of 20°C for two hours, and then adding the active first and subsequently the preservative under moderate mixing. Finally the solution is filtered aseptically through a 0.2 µm filter. Primary packaging are e.g. sterile bottles for multiple dosage:

| | |
|---|---|
| Brivudine | 0.1 % |
| Kollidon 25 | 2% |
| Boric acid | 1.5% |
| Chlorhexidin acetate | 0.03% |
| 0.1n NaOH | up to pH= 6.5 |
| Sodium-EDTA | 0.01% |
| Water q.s. | ad 100% |

### Example 2 - The eye drops formulation was prepared according to a procedure similar to example 1

| | |
|---|---|
| Brivudine | 0.1 % |
| Povidone K 25 | 5% |
| NaCl | 0.7% |
| Chlorbutanol | 0.5% |
| 0.1n NaOH | up to pH= 5.5 |
| Sodium-EDTA | 0.01% |
| Propyl gallate | 0.002% |
| Water q.s. | ad 100% |

### Example 3 - The eye drops formulation was prepared according to a procedure similar to example 1

| | |
|---|---|
| Brivudine | 0.1 % |
| Polyvinyl alcohol 72000 | 0.3% |
| Boric acid | 1.8% |
| Chlorhexidin acetate | 0.02% |
| Chlorbutanol | 0.3% |
| 0.1 n NaOH | up to pH= 5.5 |
| Sodium-EDTA | 0.01% |
| Butylated hydroxytoluene | 0.001% |
| Water q.s. | ad 100% |

### Example 4 - The eye drops formulation was prepared according to a procedure similar to example 1

| | |
|---|---|
| Brivudine | 0.1 % |
| Carbomer 980 | 6% |
| Boric acid | 1.8% |
| Chlorhexidin acetate | 0.02% |
| Chlorbutanol | 0.3% |
| 0.1n NaOH | up to pH= 5.5 |
| Sodium-EDTA | 0.01% |
| Water q.s. | ad 100% |

### Example 5 - The eye drops formulation was prepared according to a procedure similar to example 1; the primary packaging in this case (not preserved formulation) are single dose containers

| | |
|---|---|
| Brivudine | 0.1% |
| Povidone K 25 | 1.4% |
| Na₂HPO₄ | 0.65% |
| NaH₂PO₄ | 0.18% |
| NaCl | 0.23% |
| Sodium-EDTA | 0.01% |
| Water q.s. | ad 100% |

### Example 6 - The eye drops formulation was prepared according to a procedure similar to example 1

| | |
|---|---|
| Brivudine | 0.3% |
| Povidone K 25 | 2% |
| Poloxamer 407 | 10% |
| Poloxamer 188 | 15% |
| Boric acid | 1.5% |
| Chlorhexidin acetate | 0.03% |
| 0.1n NaOH | up to pH= 6.5 |
| Sodium-EDTA | 0.01% |
| Water q.s. | ad 100% |

### Example 7 - Photostability test

**Table 1: Exemplary recovery rate of brivudine in film former (PVP) containing solution (25 Wh/m² = 8.9 J/cm² (UVA/VIS) ≅ 67 min)**

| | recovered brivudine [%] | |
|---|---|---|
| Irradiation time (min) | Formulation without film former | formulation corresponding to example 1 |
| 0 | 99.8 | 101.5 |
| 10 | 96,7 | 99.8 |
| 20 | 87.9 | 98 |
| 30 | 77.4 | 93.5 |
| 67 | 33.4 | 85.5 |

Table 1 shows the brivudine amount in formulations with and without film forming agent depending on the irradiation time. Here can be observed that after irradiation of one hour only 33.4% of the starting amount of brivudine is recovered if no film former is contained.

However, it can be seen that the film forming agent causes a significant stabilisation against light induced degradation.

The photo stability tests themselves have been performed using a SUNTEST CPS + instrument of the company Atlas. The applied irradiation energy is indicated below:
Irradiation energy (time): 1.3 J/cm² (10 min), 2.6 J/cm² (20 min), 4.0 J/cm² (30 min), 8.9 J/cm² (67 min), 26.7 J/cm²(20 min)
Wave length range: 320-800 nm

Table 2 reports a direct comparison between some representative formulations containing 2% film forming agent and a formulation without film former. Just after approx. one hour the protected formulation contains 50% more brivudine than the unprotected drug.

In Table 3 the recovery of brivudine in formulation containing alternative film-forming agents is shown: These film-forming agents did not appear as satisfactory solution for the present invention.

Finally Table 4 reports the results of an intra ocular bioavailability study in rabbit. The presence of the active substance in the cornea was observed even after 6 hours. In contrast, the active was never found in blood.

The results of this study indicate that the formulation should be applied only 4 to 5 times daily and that the active is not absorbed systemically.

**Table 2: Comparison of the brivudine photo stability in formulations with and without film forming agent (PVP)**

| | recovered brivudine [%] | | | | |
|---|---|---|---|---|---|
| irradiation energy [J/cm²] | formulation corresponding to example 1 (PVP Chlorhexidin) [%] | formulation corresponding to example 2 (PVA, Chlorbutanol Chlorhexidin) | formulation corresponding to example 6 (PVP, without preservative) [%] | formulation corresponding to example 3 (PA, Chlorhexidin) [%] | formulation containing no film former [%] |
| 0[J/cm²] | 101.5 | 100.2 | 100.4 | 99 | 99.8 |
| 8.9 [J/cm²] - 67 min irradiation | 85.5 | 83.8 | 87.3 | 83.3 | 33.4 |

**Table 3: Comparison of the brivudine photo stability in formulations containing alternative film forming agents**

| | recovered brivudine [%] | | | |
|---|---|---|---|---|
| irradiation energy [J/cm²] | formulation containing agar gel as thickener | formulation containing hydroxyl propylcellulose | formulation containing sodium alginate | formulation containing carrageenan |
| 0[J/cm²] | 100,8 | 100.3 | 102.4 | 101,1 |
| 8.9 [J/cm²] - 67 min - 67 min irradiation | 35,7 | 70,7 | 33,8 | 24,0 |

### Biological test

**Table 4: Brivudine (BVDU) mean concentration in the cornea, in the aqueous humour and in blood plasma after application of formulation corresponding to example 5**

| Number of Animals | Time | BVDU in rabbit cornea **[ng/cornea]** | BVDU in rabbit aqueous humour **[ng/ml]** | BVDU in rabbit blood plasma **[ng/0.5 ml]** |
|---|---|---|---|---|
| 4 | 15 min | 36.15 | 23.35 | 0 |
| 4 | 30 min | 21.1 | 30.97 | 0 |
| 4 | 60 min | 57.57 | 59.55 | 0 |
| 4 | 2 h | 11.99 | 19.62 | 0 |
| 4 | 4 h | 3.81 | 5.6 | 0 |
| 4 | 6 h | 2.69 | 0 | 0 |

## Claims

1. Ophthalmic composition containing from 0.05 to 0.3% w/w of brivudine as active ingredient and a film-forming agent selected from the group consisting of polyvinyl pyrrolidone (PVP), polyvinyl alcohol (PVA) and polyacrylate (PA).

2. A composition according to claim 1, in which the amount of the film-forming agent ranges from 0.5 to 10% w/w.

3. A composition according to claim 2, in which said amount ranges from 1 to 5% w/w.

4. A composition according to claim 1, further containing a preservative agent in amounts up to 2% w/w.

5. A composition according to claim 4, in which said amount ranges from 0.01 to 1.5% w/w.

6. A composition according to claim 1, further containing an antioxidant agent in amounts from 0.001 to 12% by weight.

7. A composition according to claim 6, in which said amount is from 0.001 to 0.1% w/w.

8. A composition according to claim 1, which is in form of eye-drops, solution, gel, ointment.

9. A composition according to claim 8, which is packaged in single dose or multiple dose containers.

10. A composition according claim 1, containing 0.1-0.3% w/w brivudine; 1-7% w/w film-forming agent; 0-2% w/w preserving agents ; 0-2% buffer; 0-0.9% NaCl; 0-4% 0.1N NaOH to give a pH ranging from 5.5 to 6.5; 0-25% surfactant; 0-0.003% antioxidant; 0.01-0.05% Sodium-EDTA; ad 100% water.

11. A combined preparation containing an ophthalmic composition in accordance with claims 1-10 and a steroidal or non-steroidal antiinflammatory drug for simultaneous, sequential or separate use in the treatment of ocular diseases.

12. A combined preparation according to claim 11, wherein the steroid is prednisolone acetate or fluorometholone and the non-steroidal antinflammatory drug is flurbiprofen.

13. The use of an ophthalmic composition according to claims 1-10 for the preparation of a medicament for the treatment of HSV-epithelial keratitis, HSV-stromal keratitis, ocular complications of herpes zoster ophthalmicus.

14. The use of a film-forming agent selected from the group consisting of polyvinyl pyrrolidones (PVP), polyvinyl alcohol (PVA), polyacrylates (PA), for the preparation of an ophthalmic composition according to claim 1.

## Patentansprüche

1. Ophthalmische Zusammensetzung, die 0,05 bis 0,3% w/w an Brivudin als aktivem Bestandteil und ein filmbildendes Mittel, ausgewählt aus der Gruppe, bestehend aus Polyvinylpyrrolidon (PVP), Polyvinylalkohol (PVA) und Polyacrylat (PA), enthält.

2. Zusammensetzung gemäß Anspruch 1, wobei die Menge des filmbildenden Mittels von 0,5 bis 10% w/w reicht.

3. Zusammensetzung gemäß Anspruch 2, wobei die Menge von 1 bis 5% w/w reicht.

4. Zusammensetzung gemäß Anspruch 1, die ferner ein Konservierungsmittel in Mengen von bis zu 2% w/w enthält.

5. Zusammensetzung gemäß Anspruch 4, wobei die Menge von 0,01 bis 1,5% w/w reicht.

6. Zusammensetzung gemäß Anspruch 1, die ferner ein Antioxidationsmittel in Mengen von 0,001 bis 12 Gew.-% enthält.

7. Zusammensetzung gemäß Anspruch 6, wobei die Menge 0,001 bis 0,1% w/w beträgt.

8. Zusammensetzung gemäß Anspruch 1, die in der Form von Augentropfen, einer Lösung, einem Gel, einer Salbe vorliegt.

9. Zusammensetzung gemäß Anspruch 8, die in Einzeldosis- oder Mehrfachdosen-Behältern gepackt ist.

10. Zusammensetzung gemäß Anspruch 1, die 0,1-0,3% w/w Brivudin, 1-7% w/w filmbildendes Mittel, 0-2% w/w Konservierungsmittel, 0-2% Puffer, 0-0,9% NaCl, 0-4% 0,1N NaOH, um einen pH-Wert zu ergeben, der von 5,5 bis 6,5 reicht, 0-25% Tensid, 0-0,003% Antioxidationsmittel, 0,01-0,05% Natrium-EDTA, Wasser auf 100% enthält.

11. Kombiniertes Präparat, das die ophthalmische Zusammensetzung gemäß Ansprüchen 1-10 und ein steroidales oder nicht steroidales Antiphlogistikum enthält, für eine gleichzeitige, aufeinanderfolgende oder separate Verwendung bei der Behandlung von Augenkrankheiten.

12. Kombiniertes Präparat gemäß Anspruch 11, wobei das Steroid Prednisolonacetat oder Fluormetholon ist und das nicht steroidale Antiphlogistikum Flurbiprofen ist.

13. Verwendung einer ophthalmischen Zusammensetzung gemäß Ansprüchen 1-10 zur Herstellung eines Medikaments zur Behandlung von HSV-epithelialer Keratitis, HSV-stromaler Keratitis, Augenkomplikationen von Herpes zoster ophthalmicus.

14. Verwendung eines filmbildenden Mittels, ausgewählt aus der Gruppe, bestehend aus Polyvinylpyrrolidonen (PVP), Polyvinylalkohol (PVA), Polyacrylaten (PA), zur Herstellung einer ophthalmischen Zusammensetzung gemäß Anspruch 1.

## Revendications

1. Composition ophtalmique contenant de 0.05 à 0.3% poids/poids de brivudine comme ingrédient actif et un agent filmogène sélectionné dans le groupe constitué de polyvinyl pyrrolidone (PVP), alcool de polyvinyle (PVA) et polyacrylate (PA).

2. Une composition selon la revendication 1, dans laquelle la quantité d'agent filmogène varie de 0.5 à 10% poids/poids.

3. Une composition selon la revendication 2, dans laquelle ladite quantité varie de 1 à 5 % poids/poids.

4. Une composition selon la revendication 1, contenant en outre un agent de conservation dans des quantités allant jusqu'à 2% poids/poids.

5. Une composition selon la revendication 4, dans laquelle ladite quantité varie de 0.01 à 1.5% poids/poids.

6. Une composition selon la revendication 1, contenant en outre un agent antioxydant en quantités de 0.001 à 12% en poids.

7. Une composition selon la revendication 6, dans laquelle ladite quantité est de 0.001 à 0.1% poids/poids.

8. Une composition selon la revendication 1, qui est sous forme de collyre, solution, gel, pommade.

9. Une composition selon la revendication 8, qui est conditionnée en dose simple ou en récipients multi doses.

10. Une composition selon la revendication 1, contenant 0.1-0.3% poids/poids de brivudine ; 1-7% poids/poids d'agent filmogène ; 0-2% poids/poids d'agents de conservation ; 0-2% de tampon ; 0-0.9% de NaCl ; 0-4% de NaOH 1N pour donner une gamme de pH de 5.5 à 6.5 ; 0-25% de surfactant ; 0-0.003% d'antioxydant ; 0.01-0.05% de Sodium-EDTA ; qsp 100% d'eau.

11. Une préparation combinée contenant une composition ophtalmique en accord avec les revendications 1-10 et un médicament anti-inflammatoire stéroïdien ou non stéroïdien, pour une utilisation simultanée, séquentielle ou séparée dans le traitement des maladies oculaires.

12. Une préparation combinée selon la revendication 11, dans laquelle le stéroïde est l'acétate de prednisolone ou la fluorométholone et le médicament anti-inflammatoire non stéroïdien est le flurbiprofen.

13. L'utilisation d'une composition ophtalmique selon les revendications 1-10 pour la préparation d'un médicament pour le traitement de kératite épithéliale à HSV, de kératite stromale à HSV, de complications oculaires de zona ophtalmique.

14. L'utilisation d'un agent filmogène sectionné dans le groupe constitué de polyvinyl pyrrolidone (PVP), alcool de polyvinyle (PVA), polyacrylate (PA), pour la préparation d'une composition ophtalmique selon la revendication 1.
